# EUROPEAN PATENT APPLICATION

(11) **EP 4 338 744 A1**
(43) Date of publication of application: **20.03.2024**
(21) Application number: 21941384.6
(22) Date of filing: 14.05.2021
(51) Int. Cl.: A61K 31/7016, A61K 31/702, A61P 13/12

(54) **APPLICATION OF ALGINATE OLIGOSACCHARIDE**

(71) Applicant: Haitang (Jiangsu) Biotechnology Company, Ltd., Nantong, Jiangsu 226126 (CN)
(72) Inventor: LIU, Zhende, Shanghai 201201 (CN); GAO, Heyong, Shanghai 201201 (CN)
(74) Representative: Potter Clarkson
(86) International application number: PCT/CN2021/093850
(87) International publication number: WO 2022/236814

(57) **Abstract**

The present invention provides an alginate oligosaccharide or a pharmaceutically acceptable salt thereof in preparation of a drug for treatment of acute kidney injury, wherein the alginate oligosaccharide is an alginate disaccharide, an alginate trisaccharide, or an alginate tetrasaccharide. Studies have found that the alginate disaccharide, the alginate trisaccharide, and the alginate tetrasaccharide all show significant protective effects on animal models of acute kidney injury induced by ischemia-reperfusion (I/R), endotoxin phosphorus lipopolysaccharides (LPS) and anti-tumor drug cisplatin. After the treatment of acute kidney injury animals by the alginate oligosaccharide of the present invention, the level of serum creatinine decreases significantly, the urine concentration ability of kidneys recovers significantly, the level of renal tubular injury markers (KIM-1 and NGAL) is significantly reduced, the protein expression of inflammatory factors is significantly reduced, the pathological changes of the kidneys are significantly ameliorated, and the therapeutic effect is enhanced with the increase of dose. Therefore, the alginate oligosaccharide of the present invention has a strong effect in the treatment of acute kidney injury.

## Description

### FIELD OF THE INVENTION

The present invention relates to an application of an alginate oligosaccharide, belonging to the field of biomedical technologies.

### BACKGROUND OF THE INVENTION

Carbohydrates, together with nucleic acids and proteins, are called the three major living substances. Alginate, mainly found in the cell walls of kelp, sargassum and giant kelp, is a class of straight-chain, unbranched, negatively charged polysaccharide compounds. The alginate is a binary linear block compound composed of β-D-(1,4)-manuurouic acid (M) and α-L-(1,4)-guluronic acid (G). There are three main structural fragments existing in its molecules, including: polymannuronate (PM) composed of β-D-(1,4)-mannuronic acids linked to each other; polyguluronate (PG) composed of α-L-(1,4)-guluronic acids linked to each other; PMG fragments formed by alternating copolymerization of M and G.

Due to its characteristics such as high viscosity and gel-forming characteristics, the alginate has been widely used as a coagulant, a thickener, a stabilizer or the like in food, chemicals, pharmaceuticals, textile, and other industrial production. In the field of medicine, the alginate has a wide range of applications in medical biomaterials and drug sustained/controlled-release materials due to its unique physicochemical properties and good biocompatibility. Studies have also found that the alginate shows antioxidant, immunomodulatory, anti-tumor and other biological activities, but is greatly restricted in applications due to its low absorbability caused by large molecular weight and strong gelability. Oligosaccharides have aroused people's attention due to their definite structure, significant activity, good absorbability, and slight side effects.

Alginate-derived oligosaccharides, due to their unique structure, have become a hot spot in the study of carbohydrate drugs in terms of activity study in recent years, and important progress has been made in the study of their biological activity. Studies have found that the alginate-derived oligosaccharides and their derivatives have a variety of biological activities, such as antioxidant, antitumor, anticoagulant, immunomodulatory, neuroprotective, anti-inflammatory, antiviral, anti-Alzheimer's, anti-lithangiuria, and anti-diabetic activities.

Carbohydrates are a class of highly complex and varied biological macromolecules. Unlike oligonucleotides and polypeptides, carbohydrates are not just linear oligomers, but are typically branched. 9 kinds of common monosaccharides found in mammalian cells can be linked into more diverse structures than those linked by 20 kinds of natural amino acids or 4 kinds of nucleotides. This complexity of the carbohydrate structure makes it very difficult to derive pure carbohydrates from natural sources. Regardless of chemical cleavage or enzymatic cleavage, it is difficult to obtain oligosaccharides or polysaccharides with a uniform degree of polymerization by means of isolation. So far, almost all the oligosaccharides or polysaccharides used in studies are mixtures of a series of carbohydrates with an approximate degree of polymerization, leading to great challenges to the studies on their activity, metabolism and toxicology and on drug quality.

In view of the challenges in the current studies on carbohydrates, the inventor carried out previous studies to develop a series of highly specific alginate lyases capable of decomposing the alginate into disaccharides, trisaccharides or tetrasaccharides with high purity, conjugated double bonds at the non-reduced terminals, and a uniform degree of polymerization, respectively. After enzyme inactivation, the alginate disaccharides, trisaccharides or tetrasaccharides were centrifuged to collect the supernatant, which was concentrated, and then further purified through gel column or ion exchange resin to obtain the alginate disaccharides, trisaccharides or tetrasaccharides with a uniform degree of polymerization. The alginate disaccharides comprise ΔG and/or ΔM and a combination of these two structures at any ratio; the alginate trisaccharides comprise ΔGG, ΔGM, ΔMM and ΔMG as well as a combination of these four structures at any ratio; and the alginate tetrasaccharides comprise ΔGGG, ΔGGM, ΔGMG, ΔGMM, ΔMMG, ΔMMM, ΔMGG and ΔMGM as well as a combination of these eight structures at any ratio. All the oligosaccharides are monosaccharides linked by glucosidic bonds at positions 1,4; G represents α-L-guluronic acid; M represents β-D-mannuronic acid; and Δ represents that β-elimination occurs at positions 4,5 of the α-L-guluronic acid and/or β-D mannuronic acid to generate unsaturated monosaccharides with conjugated double bonds at positions 4,5 of non-reduced terminals. The structures of respective monosaccharides are shown in the figure below:

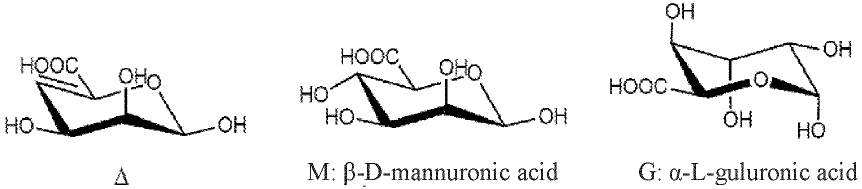

Taking ΔGM as an example, the alginate trisaccharide accordingly has a structure as follows:

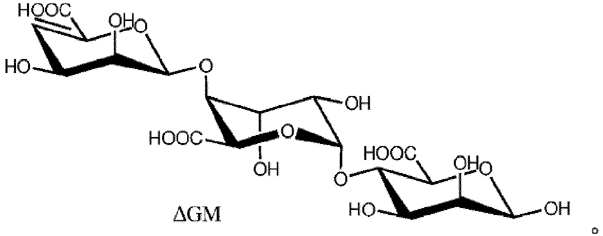

Acute kidney injury (AKI), formerly known as acute renal failure, refers to a clinical syndrome caused by rapid decline in kidney function in a short period of time due to a variety of causes. It is manifested as a rapid increase in serum creatinine and a decrease in urine output. According to the statistics, there are about 10%-20% of hospitalized patients, and even more than 50% of ICU patients, diagnosed with AKI around the world every year. Among them, 85% of patients are from developing countries. AKI may lead to increased inpatient mortality, lengthened hospital stays, increased treatment cost, and increased risk of cardiovascular events, long-term chronic kidney disease (CKD), and end-stage renal disease (ESRD). Although the nephrology community pays increasing attention to AKI, there is still no specific treatment currently available, and its incidence and mortality are still very high. Among AKI patients, the incidence of cardiovascular events is 38%. For example, the risk of heart failure is increased by 58%, the risk of acute myocardial infarction is increased by 40%, the risk of hypertension is increased by 22%, and the risk of stroke is increased by 15%, etc. AKI has become a worldwide public health issue threatening human health.

Currently, there are no effective drugs reversing AKI-induced kidney injury. Unlike some secondary CKDs such as hypertensive nephropathy, AKI involves a primary lesion in renal parenchyma (glomeruli, renal tubules and interstitium, etc.), with complex etiological factors (such as ischemia, hypoxia, toxins, drugs, and infections) and rapid progresses of the course. Some patients will develop CKD with complications such as cardiovascular diseases. Early diagnosis and timely intervention can minimize kidney injury to promote the recovery of kidney functions. At present, early identification and correction of reversible causes, maintenance of homeostasis, nutritional support, prevention of complications, and renal replacement therapy remain the main therapeutic strategies for AKI. However, hypertensive nephropathy is a lesion in renal blood vessels caused by long-term increase in blood pressure, with manifestations such as thickened renal capillaries, glomerular fibrosis, narrowed vascular lumens, renal arteriosclerosis, ischemia of renal parenchyma, and reduced nephrons. Changes in the renal parenchyma may lead to a decrease in blood filtration in the kidney and a decline in kidney function. This is a long-term secondary kidney disease caused by a persistent increase in blood pressure, and blood pressure control is the basic therapeutic strategy. Current therapies for hypertensive nephropathy mainly include blood pressure control, which, however, is not suitable for the treatment of AKI. In light of the complex etiological factors of AKI, it is highly challenging to identify a monotherapy that benefits all patients with AKI.

Based on further studies on alginate oligosaccharide, the present invention provides an application of the alginate oligosaccharide in treatment of acute kidney injury.

### SUMMARY OF THE INVENTION

Hence, an object of the present invention is to provide an application of alginate oligosaccharide.

It is achieved by the following technical solutions.

In one aspect, the present invention provides use of an alginate oligosaccharide or a pharmaceutically acceptable salt thereof in preparation of a drug for treatment of acute kidney injury, wherein the alginate oligosaccharide is an alginate disaccharide, an alginate trisaccharide, or an alginate tetrasccharide.

In some embodiments of the present invention, the alginate oligosaccharide is composed of monosaccharides G, M and/or Δ linked by glycosidic bonds at positions 1,4; and wherein G represents α-L-guluronic acid, M represents β-D-mannuronic acid, and Δ represents that β-elimination occurs at positions 4,5 of the α-L-guluronic acid or β-D mannuronic acid to generate unsaturated monosaccharides with conjugated double bonds at positions 4,5.

In some embodiments of the present invention, the alginate disaccharide is selected from ΔG, ΔM or a combination thereof.

In some embodiments of the present invention, the alginate trisaccharide is selected from one or more of ΔGG, ΔGM, ΔMM and ΔMG.

In some embodiments of the present invention, the alginate tetrasaccharide is selected from one or more of ΔGGG, ΔGGM, ΔGMG, ΔGMM, ΔMMG, ΔMMM, ΔMGG, and ΔMGM.

In some embodiments of the present invention, the pharmaceutically acceptable salt is a sodium salt, a potassium salt, a calcium salt, a magnesium salt, and/or an ammonium salt.

In some embodiments of the present invention, the acute kidney injury is induced by hypoperfusion, infection, or renal toxicity of a drug.

In another aspect, the present invention provides an alginate oligosaccharide or pharmaceutically acceptable salt thereof for treatment of acute kidney injury, wherein the alginate oligosaccharide is an alginate disaccharide, an alginate trisaccharide, or an alginate tetrasaccharide.

In further another aspect, the present invention provides an option for treating acute kidney injury, comprising administering a therapeutically effective amount of an alginate oligosaccharide or its pharmaceutically acceptable salt to a patient in need thereof, wherein the alginate oligosaccharide is an alginate disaccharide, an alginate trisaccharide, or an alginate tetrasaccharide.

The alginate disaccharide, trisaccharide and tetrasaccharide with a uniform degree of polymerization of the present invention show revolutionary progress for the quality control, the analytic investigations in pharmacology and toxicology or the like of carbohydrate-derived active pharmaceutical ingredients.

Studies have found that the alginate disaccharide, the alginate trisaccharide, and the alginate tetrasaccharide all show significant protective effects on animal models of acute kidney injury induced by ischemia-reperfusion (I/R), endotoxin phosphorus lipopolysaccharides (LPS) or antitumor drug cisplatin. In animals with acute kidney injury, after the treatment with the alginate oligosaccharide in the present invention, the level of serum creatinine decreases significantly, the urine concentration ability of kidneys recovers markedly, the level of renal tubular injury markers (KMI-1 and NGAL) is significantly reduced, the protein expression of inflammatory factors is dramatically reduced, the pathological changes of the kidneys are significantly ameliorated, all these are in a dose-dependent pattern. Therefore, the alginate oligosaccharides in the present invention have a convincible protective effect in the treatment of kidney injury.

### BRIEF DESCRIPTION OF THE FIGURES

The embodiments of the present invention are explained in detail below in conjunction with the accompanying figures, in which:
FIG. 1 shows a high-performance liquid chromatography (HPLC) of alginate disaccharides at 230 nm;
FIG. 2 shows a nuclear magnetic resonance (NMR) hydrogen spectrum (¹HNMR, D₂O as a solvent) of alginate disaccharides;
FIG. 3 shows a high-resolution mass spectrometry (HRMS(ESI)) of alginate disaccharides;
FIG. 4 shows an HPLC chromatogram of alginate trisaccharides at 230 nm;
FIG. 5 shows a ¹HNMR spectrum ( D₂O as a solvent) of alginate trisaccharides;
FIG. 6 shows a HRMS(ESI) of alginate trisaccharides;
FIG. 7 shows an HPLC chromatogram of alginate tetrasaccharides at 230 nm;
FIG. 8 shows a ¹HNMR spectrum ( D₂O as a solvent) of alginate tetrasaccharides;
FIG. 9 shows a HRMS(ESI) of alginate tetrasaccharides;
FIG. 10 shows the effect of alginate disaccharides on the level of serum creatinine in rats with AKI induced by renal ischemia-reperfusion (I/R) injury;
FIG. 11 shows the effect of alginate disaccharides on the urine output in rats with AKI induced by renal ischemia-reperfusion (I/R) injury;
FIG. 12 shows the effect of alginate disaccharides on the mRNA levels of Kim-1 and NGAL in kidney tissues of rats with AKI induced by renal ischemia-reperfusion (I/R) injury;
FIG. 13 shows the effect of alginate disaccharides on the inflammatory factors in kidney tissues of rats with AKI induced by renal ischemia-reperfusion (I/R) injury;
FIG. 14 shows the effect of alginate trisaccharides on the level of serum creatinine in rats with AKI induced by renal ischemia-reperfusion (I/R) injury;
FIG. 15 shows the effect of alginate trisaccharides on the urine output in rats with AKI induced by renal ischemia-reperfusion (I/R) injury;
FIG. 16 shows the effect of alginate trisaccharides on the mRNA levels of Kim-1 and NGAL in kidney tissues of rats with AKI induced by renal ischemia-reperfusion (I/R) injury;
FIG. 17 shows the effect of alginate trisaccharides on the inflammatory factors in kidney tissues of rats with AKI induced by renal ischemia-reperfusion (I/R) injury;
FIG. 18 shows the effect of alginate trisaccharides on the pathological sections of kidney tissue injury in rats with AKI induced by renal ischemia-reperfusion (I/R) injury;
FIG. 19 shows the effects of alginate tetrasaccharides and mixed saccharides on the level of serum creatinine in rats with AKI induced by renal ischemia-reperfusion (I/R) injury;
FIG. 20 shows the effects of alginate tetrasaccharides and mixed saccharides on the urine output in rats with AKI induced by renal ischemia-reperfusion (I/R) injury;
FIG. 21 shows the effects of alginate tetrasaccharides and mixed saccharides on the mRNA levels of Kim-1 and NGAL in kidney tissues of rats with AKI induced by renal ischemia-reperfusion (I/R) injury;
FIG. 22 shows the effect of alginate tetrasaccharides on the inflammatory factors in kidney tissues of rats with AKI induced by renal ischemia-reperfusion (I/R) injury;
FIG. 23 shows the effect of alginate disaccharides on the level of serum creatinine in mice with AKI induced by endotoxin phosphorus lipopolysaccharides (LPS);
FIG. 24 shows the effect of alginate disaccharides on the mRNA levels of Kim-1 and NGAL in kidney tissues of mice with AKI induced by endotoxin phosphorus lipopolysaccharides (LPS);
FIG. 25 shows the effect of alginate disaccharides on the inflammatory factors in kidney tissues of mice with AKI induced by endotoxin phosphorus lipopolysaccharides (LPS);
FIG. 26 shows the effects of alginate disaccharides and reference substance dexamethasone on the inflammatory factors in kidney tissues of mice with AKI induced by endotoxin phosphorus lipopolysaccharides (LPS);
FIG. 27 shows the effect of alginate trisaccharides on the level of serum creatinine in mice with AKI induced by endotoxin phosphorus lipopolysaccharides (LPS);
FIG. 28 shows the effect of alginate trisaccharides on the mRNA levels of Kim-1 and NGAL in kidney tissues of mice with AKI induced by endotoxin phosphorus lipopolysaccharides (LPS);
FIG. 29 shows the effect of alginate trisaccharides on the inflammatory factors in kidney tissues of mice with AKI induced by endotoxin phosphorus lipopolysaccharides (LPS);
FIG. 30 shows the effects of alginate trisaccharides and reference substance dexamethasone on the inflammatory factors in kidney tissues of mice with AKI induced by endotoxin phosphorus lipopolysaccharides (LPS);
FIG. 31 shows the effect of alginate trisaccharides on the pathological sections of kidney tissue injury in mice with AKI induced by endotoxin phosphorus lipopolysaccharides (LPS);
FIG. 32 shows the effects of alginate tetrasaccharides and mixed saccharides on the level of serum creatinine in mice with AKI induced by endotoxin phosphorus lipopolysaccharides (LPS);
FIG. 33 shows the effects of alginate tetrasaccharides and mixed saccharides on the mRNA levels of Kim-1 and NGAL in kidney tissues of mice with AKI induced by endotoxin phosphorus lipopolysaccharides (LPS);
FIG. 34 shows the effects of alginate tetrasaccharides and mixed saccharides on the inflammatory factors in kidney tissues of mice with AKI induced by endotoxin phosphorus lipopolysaccharides (LPS);
FIG. 35 shows the effects of alginate disaccharides, mixed saccharides and reference substance dexamethasone on the inflammatory factors in kidney tissues of mice with AKI induced by endotoxin phosphorus lipopolysaccharides (LPS);
FIG. 36 shows the effect of alginate trisaccharides on the level of serum creatinine of mice with AKI induced by cisplatin;
FIG. 37 shows the effect of alginate trisaccharides on the urine output of mice with AKI induced by cisplatin;
FIG. 38 shows the effect of alginate trisaccharides on the mRNA levels of Kim-1 and NGAL in kidney tissues of mice with AKI induced by cisplatin;
FIG. 39 shows the effect of alginate trisaccharides on the inflammatory factors in kidney tissues of mice with AKI induced by cisplatin;
FIG. 40 shows the mass spectrum of mixed saccharides used in Examples 4 and 5 of the present invention.

### DETAILED DESCRIPTION OF THE PREFERRED EMBODIMENTS

The present invention is further explained below in conjunction with examples, which are merely for explanatory and illustrative purposes and are not intended to limit the scope of the present invention in any way.

### Example 1 Preparation of alginate disaccharides with uniform degree of polymerization and structural identification of alginate disaccharides

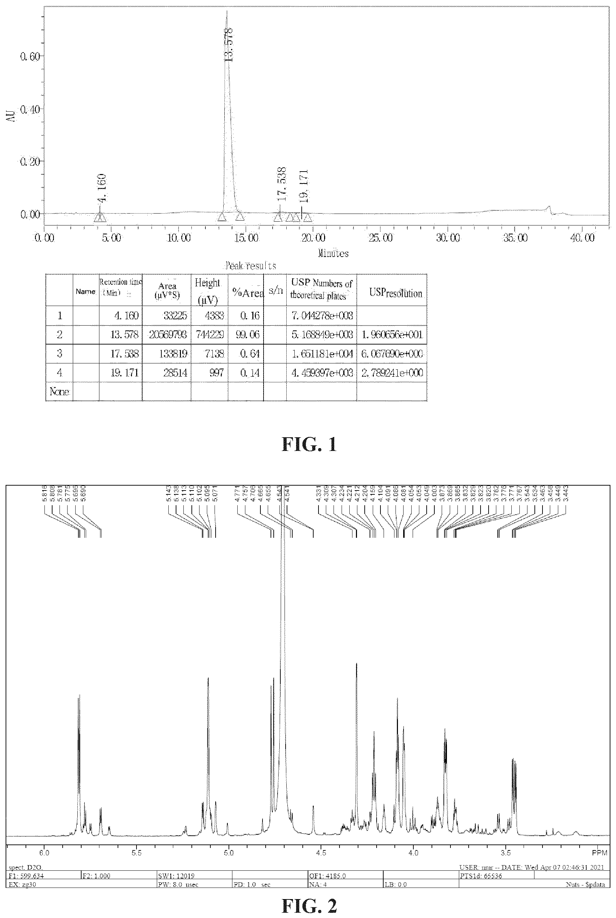

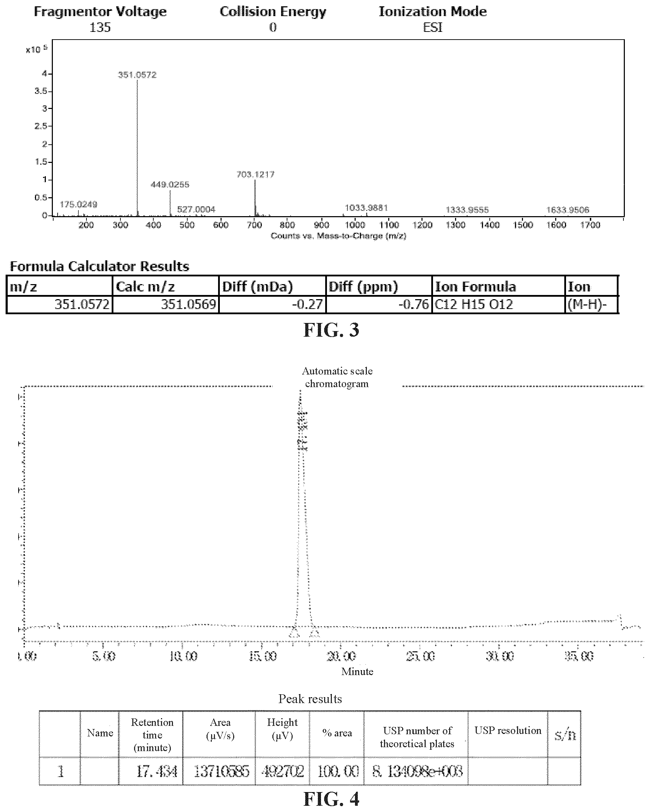

100 g of commercially available alginate (purchased from Bright Moon Seaweed Group, Qingdao, China) was dissolved in water, alginate lyases (from Ocean University of China) were added at certain temperature, and after a certain period of enzymolysis, the mixture was centrifuged with a high-speed centrifuge to collect the supernatant. The supernatant was then purified by a gel column to remove a small amount of oligosaccharide and polysaccharide impurities and non-carbohydrate impurities, and 60 g of alginate disaccharide sodium salt with a purity of more than 95% was obtained. The obtained alginate disaccharide sodium salt was detected by high-performance liquid chromatography (HPLC, 230 nm), and then structurally identified by proton magnetic resonance spectroscopy (¹HNMR) and high-resolution mass spectrometry (HRMS-ESI).
HPLC: purity 99.06%, RT=13.6 min (see FIG. 1);
¹HNMR spectrum is shown in FIG. 2;
HRMS (ESI) m/z: C₁₂H₁₅O₁₂{(M-H)⁻}, calculated for 351.0569; Found: 351.0572(M-H)⁻. (See FIG. 3);

For the alginate disaccharide sodium salt, if both carboxyl groups in the molecule are sodium salt, the theoretical ratio of sodium ions is 11.58%. Based on actual detection by ion chromatography, the ratio of sodium ions is 10.3%. In the event of detection by residue on ignition, the sodium ions exist in the form of sodium sulfate, and the theoretical residue ratio should be 35.77%. Based on actual detection by residue on ignition, the residue accounts for 34.3%. The results obtained by the two detection methods were relatively approximate, indicating that the carboxylate functional group of the compound is indeed in the form of sodium salt. However, the measured values are both slightly less than the theoretical values, which possibly lies in that the alginate disaccharide sodium salt is a weak acid and strong base salt, and a small amount of carboxylate is still free acid.

### Example 2 Preparation of alginate trisaccharides with uniform degree of polymerization and structural identification of alginate trisaccharides

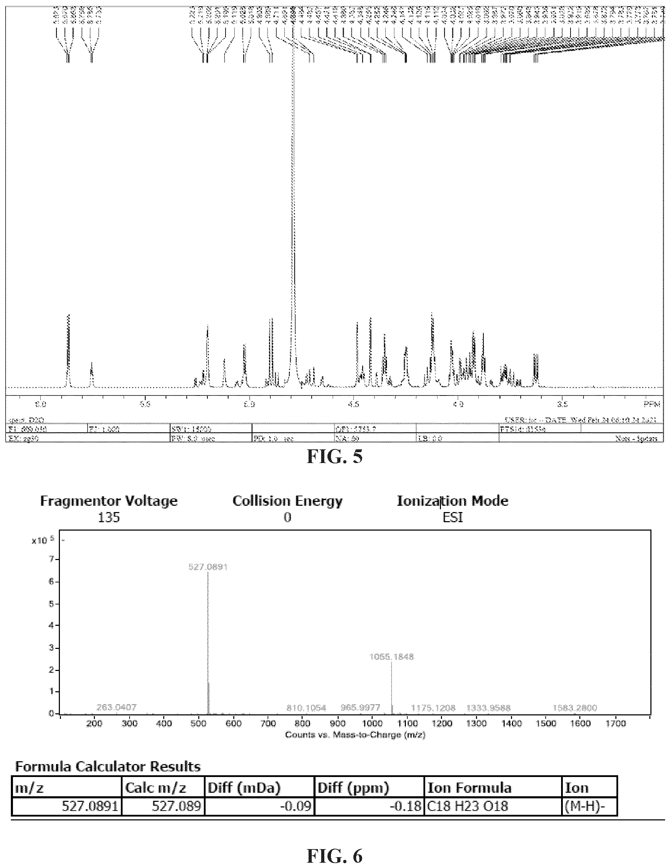

100 g of commercially available purchased alginate was dissolved in water, alginate lyases (from Ocean University of China) were added at certain temperature, and after a certain period of enzymolysis, the mixture was centrifuged with a high-speed centrifuge to collect the supernatant. The supernatant was then purified by a gel column to remove a small amount of oligosaccharide and polysaccharide impurities and non-carbohydrate impurities, and 70g of alginate trisaccharide sodium salt with a purity of more than 95% was obtained. The obtained alginate trisaccharide sodium salt was detected by HPLC (230 nm), and then structurally identified by ¹HNMR and HRMS-ESI.
HPLC: purity 100%, RT=17.43 min (see FIG. 4);
¹HNMR spectrum is shown in FIG. 5;
HRMS (ESI) m/z: C₁₈H₂₃O₁₈{(M-H)⁻}, calculated for 527.0890; Found: 527.0891 (M-H)⁻. (See FIG. 6);

For the alginate trisaccharide sodium salt, if three carboxyl groups in the molecule are all sodium salt, the theoretical ratio of sodium ions is 11.59%. Based on actual detection by ion chromatography, the ratio of sodium ions is 9.9%. In the event of detection by residue on ignition, the sodium ions exist in the form of sodium sulfate, and the theoretical residue ratio should be 35.80%; and the measured residue on ignition accounts for 33.01%. The results obtained by the two detection methods were relatively approximate, indicating that the carboxylate functional group of the compound is indeed in the form of sodium salt. However, the measured values are both slightly less than the theoretical values, which possibly lies in that the alginate disaccharide sodium salt is a weak acid and strong base salt, and a small amount of carboxylate is still free acid.

### Example 3 Preparation of alginate tetrasaccharides with uniform degree of polymerization and structural identification of alginate tetrasaccharides

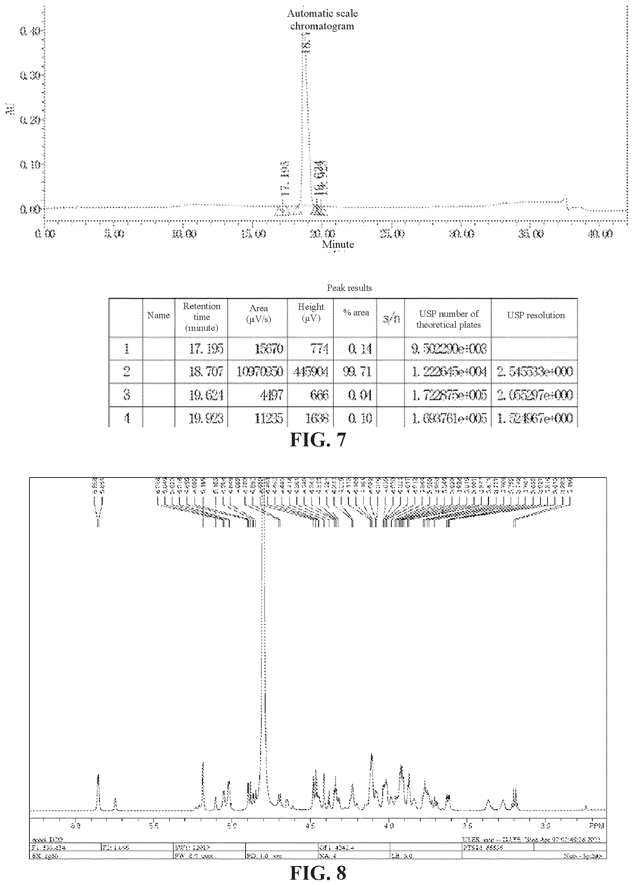

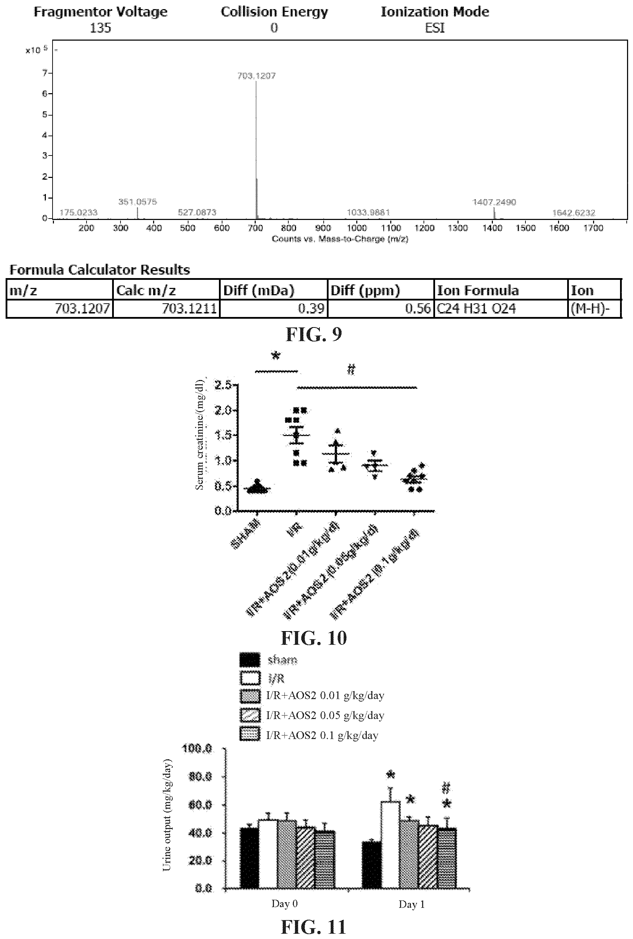

100 g of commercially available purchased alginate was dissolved in water, alginate lyases (from Ocean University of China) were added at certain temperature, and after a certain period of enzymolysis, the mixture was centrifuged with a high-speed centrifuge to collect the supernatant. The supernatant was then purified by a gel column to remove a small amount of oligosaccharide and polysaccharide impurities and non-carbohydrate impurities, and 55g of alginate tetrasaccharide sodium salt with a purity of more than 95% was obtained. The obtained alginate tetrasaccharide sodium salt was detected by HPLC (230 nm), and then structurally identified by ¹HNMR and HRMS-ESI.
HPLC: purity 99.71%, RT=18.71 min (see FIG. 7);
¹HNMR spectrum is shown in FIG. 8;
HRMS (ESI) m/z: C₂₄H₃₁O₂₄{(M-H)⁻}, calculated for 703.1211; Found: 703.1207(M-H)⁻. (See FIG. 9);

For the alginate tetrasaccharide sodium salt, if four carboxyl groups in the molecule are all sodium salt, the theoretical ratio of sodium ions is 11.59%. Based on actual detection by ion chromatography, the ratio of sodium ions is 9.89%. In the event of detection by residue on ignition, the sodium ions exist in the form of sodium sulfate, and the theoretical residue ratio should be 35.80%; and based on actual detection by residue on ignition, the residue accounts for 32.5%. The results obtained by the two detection methods were relatively approximate, indicating that the carboxylate functional group of the compound is indeed in the form of sodium salt. However, the measured values are both slightly less than the theoretical values, which possibly lies in that the alginate tetrasaccharide sodium salt is a weak acid and strong base salt, and a small amount of carboxylate is still free acid.

### Example 4 Effect of alginate oligosaccharides with uniform degree of polymerization against rat acute kidney injury (AKI) induced by ischemia-reperfusion (I/R)

Ischemia-reperfusion induced kidney injury is a classical animal model that simulates the acute kidney injury caused by clinical hypoperfusion. With the rat ischemia-reperfusion model, the author of the invention administered the alginate oligosaccharides with a uniform degree of polymerization as prepared by Example 1-3 and a mixture thereof, and then carried out comparison with a blank group and a non-administrated model group to investigate the therapeutic effects of the several alginate oligosaccharides with a uniform degree of polymerization.

### I. Effect of alginate disaccharides against rat acute kidney injury induced by ischemia-reperfusion

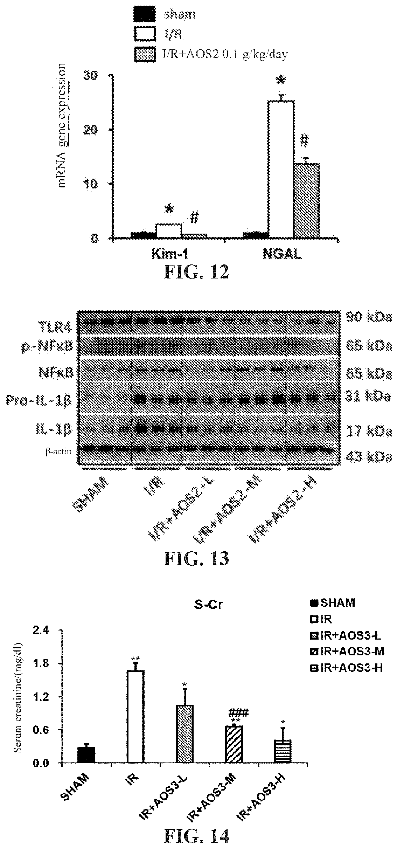

30 male Sprague Dawley rats (purchased from the Laboratory Animal Center of Sun Yat-sen University) of 220-250 grams were selected. Before treatment, the 24-hour urine output was collected and measured without abnormalities. The rats to be modeled were randomly divided into the following groups: a sham group, a model group, and three alginate disaccharide groups with the doses of 0.01 g/kg/day, 0.05 g/kg/day and 0.1 g/kg/day respectively (6 rats in each group). In the present invention, the alginate disaccharide is abbreviated as "AOS2". Intragastric administration was adopted, and rats in the model group and the sham group were all fed by gavage with the same volume of normal saline. On the day of operation, the rats were anesthetized intraperitoneally with 3% pentobarbital sodium and subjected to routine skin disinfection. The abdomen was sectioned to expose the left and right kidneys. In the sham group, the kidneys were only examined, and then the incision was sutured layer by layer to end the operation. In the model group and the modeled administration group, large bulldog clamps were used for occlusion of the renal pedicles on both sides, then the kidneys were recovered in place, and the incision was covered with gauze and dropwise added with a small amount of normal saline for rehydration. After 45 minutes, the bulldog clamps on both sides were released, and the incision was sutured layer by layer to end the operation. After operation, the rats were placed on a heating pad at 37°C to wait for resuscitation, and then placed back into metabolic cages, in which the weight, food intake, water intake and urine output of the rats were measured. The rats were routinely fed after operation and sacrificed 24 hours later for sampling. Blood samples were collected from the inferior vena cava of rats, and then centrifuged to collect upper serum. Then, the serum creatinine in the rats was determined by using the creatinine assay kit from Nanjing Jiancheng, with the results shown in FIG. 10. The results of 24-hour urine output are shown in FIG. 11. During sampling, the kidneys on both sides were isolated, the cortex and inner medulla of each kidney were isolated, and the cortex was stored in trizol. When in use, mRNAs in the cortex were extracted using the trizol method, and the mRNA expressions of AKI biomarkers (KIM-1 and NGAL) were detected. The results are shown in FIG. 12. A tissue lysis buffer was added to homogenize the kidneys by ultrasound, the total protein was then extracted, and the inflammatory factors (p-NFκB/NFκB and pro-IL-1β/ IL-1β) in the renal cortex tissues were detected by western blotting. The results are shown in FIG. 13. The experimental data were statistically analyzed by one-way ANOVA.

FIG. 10 showed that the acute ischemia-reperfusion (I/R) injury induced a significant increase in the level of serum creatinine in rats, and different doses of AOS2 reduced the serum creatinine to varying degrees, suggesting that AOS2 had a renoprotective effect.* indicates p<0.05 as compared to the sham (the sham group), and # indicates p<0.05 as compared to the I/R group (the model group).

The results showed that the decreased filtration occurred in the kidneys of rats in the I/R group after the ischemia-reperfusion injury, and the serum creatinine was significantly increased. The serum creatinine decreased significantly after treatment with AOS2 at three different doses, suggesting a recovery of the glomerular filtration. In particular, when the dose of administration was 0.1 g/kg/day, the serum creatinine could basically return to a normal level, indicating that AOS2 had a significant therapeutic effect on acute kidney injury, and its therapeutic effect showed a certain degree of dose dependence.

FIG. 11 showed that the acute ischemia-reperfusion (I/R) injury induced an increase in urine output of the rats, and different doses of AOS2 reduced the urine output of rats, suggesting that AOS2 had a renoprotective effect.* indicates p<0.05 as compared to the sham, and # indicates p<0.05 as compared to the I/R group. The results showed that impaired urine concentration occurred in the kidneys of rats in the I/R group after the ischemia-reperfusion injury, and the urine output was significantly increased. The urine output decreased somewhat after treatment with AOS2 at three different doses, suggesting certain recovery of the renal tubular reabsorption function.

FIG. 12 showed that the acute ischemia-reperfusion (I/R) injury induced an increase in the mRNA levels of acute kidney injury (AKI) markers s (i.e., renal tubular injury markers) Kim-1 and NGAL in rat kidney tissues, and AOS2 at a dose of 0.1 g/kg/day significantly reduced the expressions of both markers, suggesting that AOS2 had a renoprotective effect. * indicates p<0.05 as compared to the sham, and # indicates p<0.05 as compared to the I/R group. The results showed that the expressions of acute kidney injury (AKI) markers KIM-1 and NGAL were significantly increased in the rats in the I/R group after the ischemia-reperfusion injury, indicating renal tubular injury, and AOS2 at a dose of 0.1 g/kg/day could significantly reduce both markers, indicating that AOS2 had a significant protective effect against the acute kidney injury induced by the ischemia-reperfusion.

FIG. 13 showed that the acute ischemia-reperfusion (I/R) injury induced a significant increase in the inflammatory factors in the rat kidney tissues, and different doses of AOS2 inhibited the inflammatory responses of the kidneys to varying degrees, suggesting that AOS2 had a renoprotective effect. The dose of I/R+AOS2-L was 0.01 g/kg/day; the dose of I/R+AOS2-M was 0.05 g/kg/day; and the dose of I/R+AOS2-H was 0.1 g/kg/day. The results showed that the renal inflammatory factors such as TLR4, p-NFκB/NFκB, and pro-IL-1β/IL-1β increased significantly in rats in the I/R group after the ischemia-reperfusion injury, and the treatment with AOS2 reduced the production of inflammatory factors and showed a certain degree of dose dependence, indicating a significant anti-inflammatory effect of AOS2.

### II. Effect of alginate trisaccharides against rat acute kidney injury induced by ischemia-reperfusion

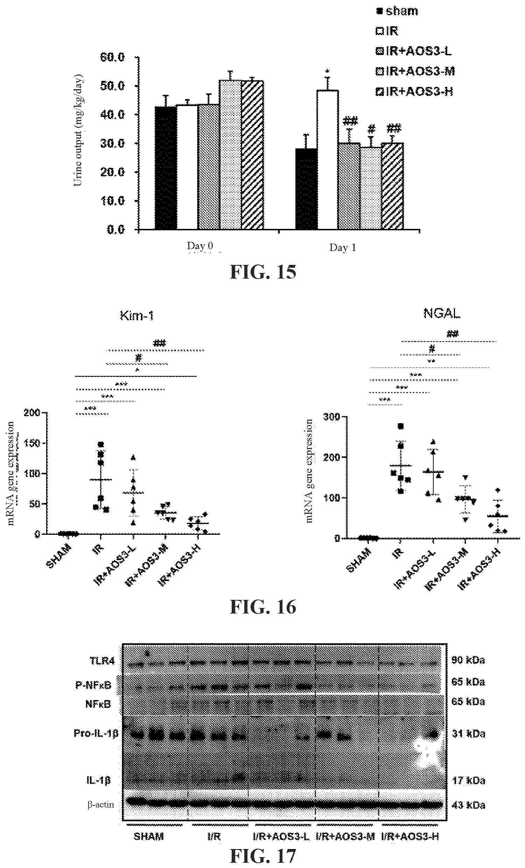

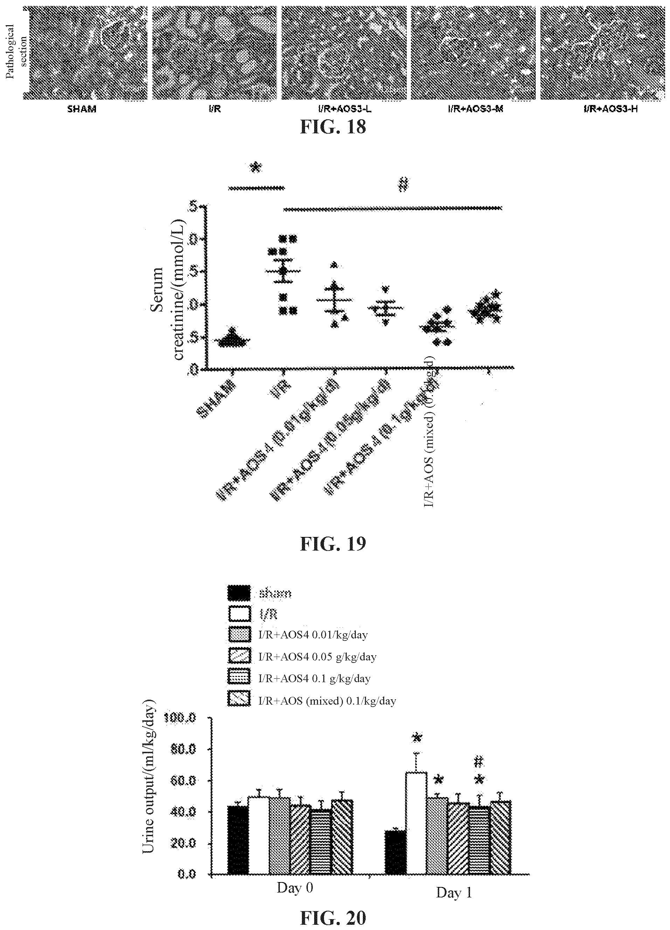

With the same experimental method as that for the alginate disaccharides, the effect of alginate trisaccharides against the rat acute kidney injury induced by ischemia-reperfusion was investigated. In the present invention, the alginate trisaccharide is abbreviated as "AOS3". The serum creatinine in the rats was determined by using the creatinine assay kit from Nanjing Jiancheng, with the results shown in FIG. 14. The results of 24-hour urine output are shown in FIG. 15. During sampling, the kidneys on both sides were isolated, the cortex and inner medulla of each kidney were isolated, and the cortex was stored in trizol. When in use, mRNAs in the cortex were extracted using the trizol method, and the mRNA expression of AKI biomarkers (KIM-1 and NGAL) was detected. The results are shown in FIG. 16. A tissue lysis buffer was added to homogenize the kidneys by ultrasound, the total protein was then extracted, and the inflammatory factors (p-NFκB/NFκB and pro-IL-1β/ IL-1β) in the renal cortex tissues were detected by western blotting. The results are shown in FIG. 17. The experimental data were statistically analyzed by one-way ANOVA. In addition, the animals were sacrificed after the last blood collection, the kidneys were fixed in 4% formaldehyde solution, and subjected to conventional paraffin embedding, sectioning, and HE staining to observe the general kidney tissue morphology under a light microscope. The results are shown in FIG. 18.

FIG. 14 showed that the acute ischemia-reperfusion (I/R) injury induced a significant increase in the level of serum creatinine in rats, and different doses of AOS3 reduced the serum creatinine to varying degrees, suggesting that AOS3 had a renoprotective effect.* indicates p<0.05 as compared to the sham, and # indicates p<0.05 as compared to the I/R group. The results showed that the decreased filtration occurred in the kidneys of rats in the I/R group after the ischemia-reperfusion injury, and the serum creatinine was significantly increased. The serum creatinine decreased significantly after treatment with AOS3 at three different doses, suggesting a recovery of the glomerular filtration. In particular, when the dose of administration was 0.1 g/kg/day, the serum creatinine could basically return to a normal level, and the therapeutic effect showed a certain degree of dose dependence.

FIG. 15 showed that the acute ischemia-reperfusion (I/R) injury induced an increase in urine output of the rats, and different doses of AOS3 reduced the urine output of rats, suggesting that AOS3 had a renoprotective effect.* indicates p<0.05 as compared to the sham, and # indicates p<0.05 as compared to the I/R group. The results showed that impaired urine concentration occurred in the kidneys of rats in the I/R group after the ischemia-reperfusion injury, and the urine output was significantly increased. After the treatment with AOS3 at three different doses, the urine output somewhat decreased in each case, especially at a dose of 0.1 g/kg/day, and the urine output of rats on the next day basically recovered to the normal level, indicating certain recovery of the kidney tubular function. The therapeutic effect was somewhat dose-dependent.

FIG. 16 showed that the acute ischemia-reperfusion (I/R) injury induced an increase in the mRNA levels of acute kidney injury (AKI) markers (i.e., renal tubular injury markers) Kim-1 and NGAL in rat kidney tissues, and AOS3 at a dose of 0.1 g/kg/day significantly reduced the expressions of both markers, suggesting that AOS3 had a renoprotective effect. * indicates p<0.05 as compared to the sham, and # indicates p<0.05 as compared to the I/R group. The results showed that the expressions of acute kidney injury (AKI) markers KIM-1 and NGAL were significantly increased in the rats in the I/R group after the ischemia-reperfusion injury, and AOS3 at a dose of 0.1 g/kg/day could significantly reduce both markers, indicating that AOS3 had a significant protective effect against the acute kidney injury induced by the ischemia-reperfusion.

FIG. 17 showed that the acute ischemia-reperfusion (I/R) injury induced a significant increase in the inflammatory factors in the rat kidney tissues, and different doses of AOS3 inhibited the inflammatory responses of the kidneys to varying degrees, suggesting that AOS3 had a renoprotective effect. I/R+ AOS3-L indicated a dose of 0.01 g/kg/day; I/R+ AOS3-M indicated a dose of 0.05 g/kg/day; and I/R+ AOS3-H indicated a dose of 0.1 g/kg/day. The results showed that the renal inflammatory factors TLR4, p-NFκB/NFκB, and pro-IL-1β/IL-1β increased significantly in rats in the I/R group after the ischemia-reperfusion injury, and the treatment with AOS3 at different doses could reduce the production of inflammatory factors and showed a certain degree of dose dependence, indicating a significant anti-inflammatory effect of alginate trisaccharides.

FIG. 18 showed the renal histopathological manifestations. Specifically, in the sham group (SHAM), the glomerular morphology, mesangial cells and renal tubules were basically normal; in the I/R group, the renal tubules were widely dilated, with enlarged lumens, a large number of epithelial cells underwent swelling, brush border loss, necrosis and shedding, and vacuolar was visible in the tubular epithelial cells; and in the I/R+AOS3 group (0.1 g/kg/day), the renal tubules showed mild lesions. These results suggested very obvious lesions in renal tubules for the rats in the I/R group. However, after the administration of AOS3 (0.1 g/kg/day) for treatment after similar ischemia-reperfusion, the kidney injury was very mild. This showed AOS3 had a good protective effect against rat renal morphological changes induced by ischemia-reperfusion.

### III. Effect of alginate tetrasaccharides and mixed saccharides against rat acute kidney injury induced by ischemia-reperfusion

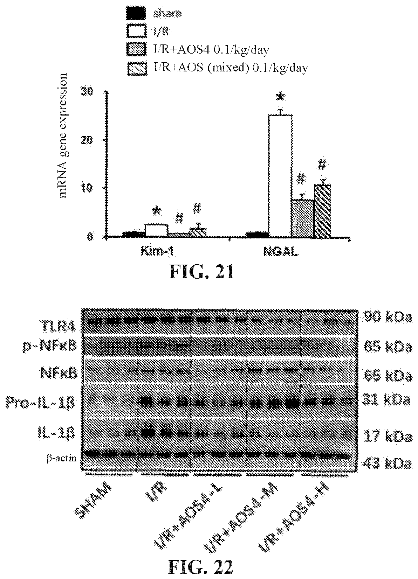

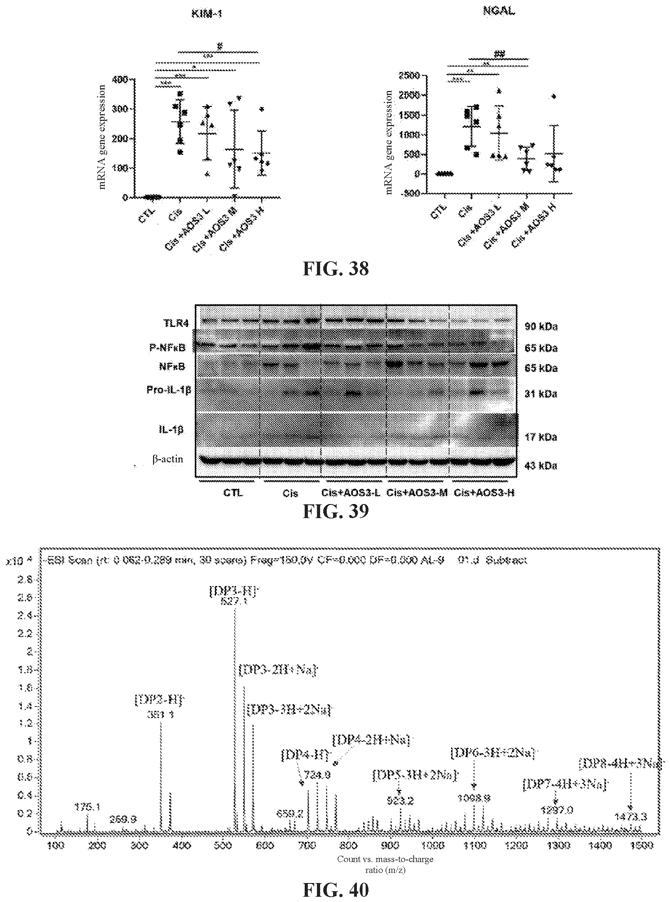

The same experimental method was used as that for the alginate disaccharides and alginate trisaccharides. The modeled rats were randomly divided into a sham group, a model group, alginate tetrasaccharides groups (at three doses of 0.01 g/kg/day, 0.05 g/kg/day, and 0.1 g/kg/day) and a mixed saccharides (including mixed alginate oligosaccharides having a degree of polymerization of 2-8, the mass spectrum of which is shown in FIG. 40, and which was obtained from Ocean University of China, at one dose of 0.1 g/kg/day) group (6 rats in each group). In the present invention, the alginate tetrasaccharides is abbreviated as "AOS4", and the mixed saccharides were abbreviated as "AOS (mixed)". The serum creatinine in the rats was determined by using the creatinine assay kit from Nanjing Jiancheng, with the results shown in FIG. 19. The results of 24-hour urine output are shown in FIG. 20. During sampling, the kidneys on both sides were isolated, the cortex and inner medulla of each kidney were isolated, and the cortex was stored in trizol. When in use, mRNAs in the cortex were extracted using the trizol method, and the mRNA expression of AKI biomarkers (KIM-1 and NGAL) was detected. The results are shown in FIG. 21. A tissue lysis buffer was added to homogenize the kidneys by ultrasound, the total protein was then extracted, and the inflammatory factors (p-NFκB/NFκB and pro-IL-1β/ IL-1β) in the renal cortex tissues were detected by western blotting. The results are shown in FIG. 22. The experimental data were statistically analyzed by one-way ANOVA.

FIG. 19 showed that the acute ischemia-reperfusion (I/R) injury induced a significant increase in the level of serum creatinine in rats, and AOS4 at different doses and the mixed saccharides reduced the serum creatinine to varying degrees, suggesting that AOS4 had a good renoprotective effect.* indicates p<0.05 as compared to the sham, and # indicates p<0.05 as compared to the I/R group. The results showed that the decreased filtration occurred in the kidneys of rats in the I/R group after the ischemia-reperfusion injury, and the serum creatinine was significantly increased. The serum creatinine decreased significantly after treatment with AOS4 at three different doses and the mixed saccharides (0.1 g/kg/day), suggesting a recovery of glomerular filtration. In particular, with the alginate tetrasaccharides administered at a dose of 0.1 g/kg/day, the serum creatinine could restore to a normal level. The therapeutic effect of AOS4 at different doses showed a certain degree of dose dependence.

FIG. 20 showed that the acute ischemia-reperfusion (I/R) injury induced an increase in urine output of the rats, and AOS4 at different doses and the mixed saccharides (0.1 g/kg/day) reduced the urine output of rats, indicating that AOS4 had a renoprotective effect. * indicates p<0.05 as compared to the sham, and # indicates p<0.05 as compared to the I/R group. The results showed that impaired urine concentration occurred in the kidneys of rats in the I/R group after the ischemia-reperfusion injury, and the urine output was significantly increased. After treatment with the AOS4 at three different doses and the mixed saccharides, the urine output was somewhat reduced, suggesting certain recovery of the renal tubule function. In particular, the therapeutic effect of AOS4 was obviously better than that of the mixed saccharides at the same dose. The therapeutic effect showed a certain degree of dose dependence.

FIG. 21 showed that the acute ischemia-reperfusion (I/R) injury induced an increase in the mRNA levels of acute kidney injury (AKI) markers (i.e., renal tubular injury markers) Kim-1 and NGAL in rat kidney tissues, and the alginate tetrasaccharides at a dose of 0.1 g/kg/day significantly reduced the expressions of both markers, suggesting that the alginate tetrasaccharides had a renoprotective effect. * indicates p<0.05 as compared to the sham, and # indicates p<0.05 as compared to the I/R group. The results showed that the expressions of acute kidney injury (AKI) markers KIM-1 and NGAL were significantly increased in the rats in the I/R group after the ischemia-reperfusion injury, and AOS4 at a dose of 0.1 g/kg/day could significantly reduce both markers, indicating that AOS4 had a protective effect against the acute kidney injury induced by the ischemia-reperfusion.

FIG. 22 showed that the acute ischemia-reperfusion (I/R) injury induced a significant increase in the inflammatory factors in the rat kidney tissues, and different doses of AOS4 could inhibit the inflammatory responses of the kidneys to varying degrees, suggesting that AOS4 had a renoprotective effect.* I/R+ AOS4-L indicated a dose of 0.01 g/kg/day; I/R+ AOS4-M indicated a dose of 0.05 g/kg/day; I/R+ AOS4-H indicated a dose of 0.1 g/kg/day; and I/R+ AOS (mixed) indicated a dose of 0.1 g/kg/day. The results showed that the renal inflammatory factors such as TLR4, p-NFκB/NFκB, and pro-IL-1β/IL-1β increased significantly in rats in the I/R group after the ischemia-reperfusion injury, and the treatment with AOS4 could significantly reduce the production of inflammatory factors and showed a certain degree of dose dependence, showing an anti-inflammatory effect of AOS4.

### Experimental conclusion

Ischemia-reperfusion induced kidney injury is a classical animal model that simulates the acute kidney injury caused by clinical hypoperfusion. After treatment by administration of alginate disaccharides, alginate trisaccharides and alginate tetrasaccharides at different doses (0.01, 0.05 and 0.1 g/kg/day), the level of serum creatinine in the rats decreased significantly, and basically returned to normal on average at the dose of 0.1 g/kg/day. After administration, the urine concentration ability of the kidneys was ameliorated significantly, with decreased urine output. Moreover, in the group treated at the dose of 0.1 g/kg/day, the levels of kidney injury factors (KIM-1 and NGAL) were significantly reduced; the expressions of inflammatory factors decreased significantly; the pathological changes of the kidneys were significantly ameliorated; and the therapeutic effect was enhanced with the increase of dose. In summary, the results in terms of serum creatinine, urine output, inflammatory factors and other different parameters show that the alginate disaccharides, trisaccharides, and tetrasaccharides all have a convincible protective effect against the acute kidney injury in rats with AKI induced by ischemia-reperfusion. Compared with the mixed alginate oligosaccharides with a degree of polymerization of 2-8, the alginate disaccharides, trisaccharides, and tetrasaccharides with a uniform degree of polymerization show an obviously better therapeutic effect.

### Example 5 Effect of alginate oligosaccharides with a uniform degree of polymerization against mouse acute kidney injury induced by endotoxin phosphorus lipopolysaccharides (LPS)

The kidney injury induced by treatment with endotoxin phosphorus lipopolysaccharides is a classical and standard animal model that simulates the acute kidney injury induced by clinical infection. With the mouse endotoxin phosphorus lipopolysaccharide model, we administered the alginate oligosaccharides with different uniform degrees of polymerization and a mixture thereof, and then compared with a blank group and a non-administrated model group to investigate the therapeutic effects of the individual alginate oligosaccharides with a uniform degree of polymerization.

### I. Effect of alginate disaccharides (AOS2) against mouse acute kidney injury induced by LPS

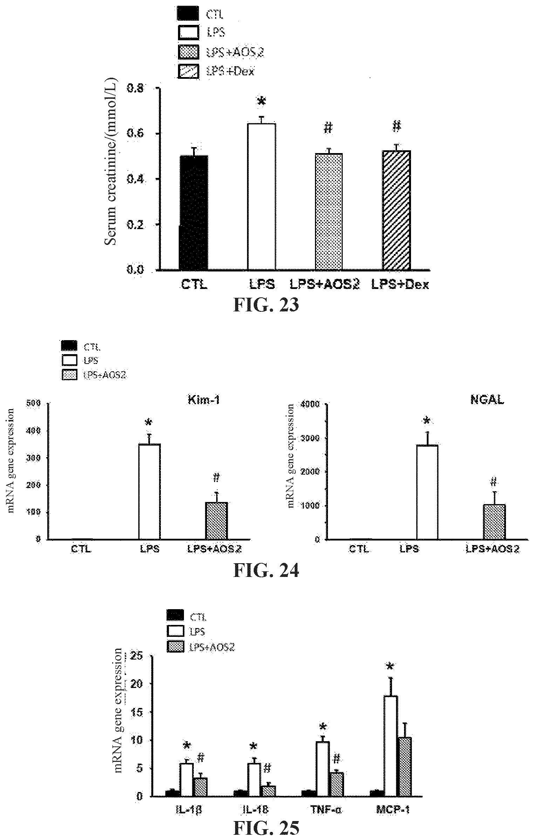

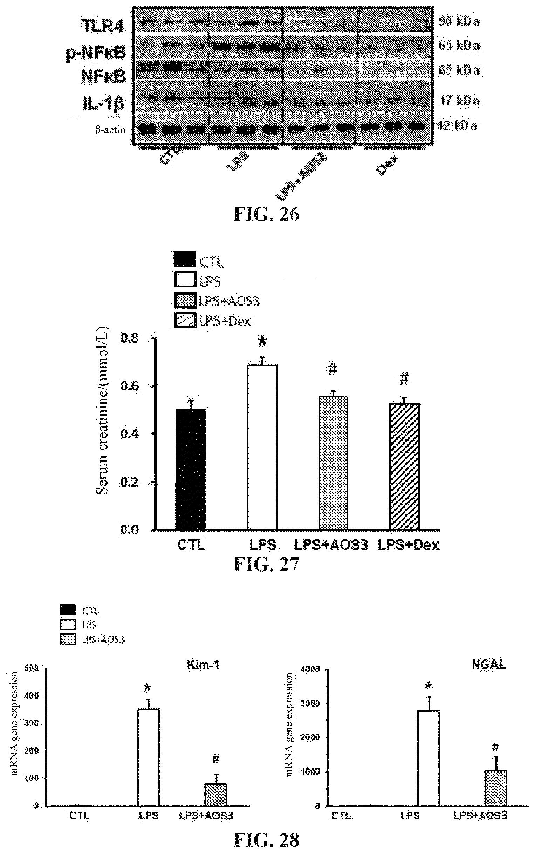

24 male C57/B16 mice of 22-28 grams were selected. Before treatment, the 24-hour urine output was collected and measured without abnormalities. The mice to be modeled were randomly divided into the following groups: a control group, a model group, a model + administration group (AOS2 at 0.1 g/kg/day), and a model + positive control group (dexamethasone acetate at 0.1 g/kg/day), with 6 mice in each group. Intragastric administration was adopted for samples; the model group and the control group were all fed by gavage with the same volume of normal saline; and dexamethasone was administrated by intraperitoneal injection. LPS was used for modeling to induce the occurrence of sepsis-associated acute kidney injury. During modeling, each model group was intraperitoneally injected with LPS at 15 mg/kg, and the control group was intraperitoneally injected with the same volume of normal saline. Immediately after modeling, the mice were put back into the mouse metabolic cages for observation, in which the weight, food intake, water intake and urine output of the mice were measured. The mice were sacrificed 24 hours later for sampling, and urine was collected. Blood samples were collected from the inferior vena cava of mice, and then centrifuged to collect upper serum. Then, the serum creatinine in the mice was determined by using the creatinine assay kit from Nanjing Jiancheng, with the results shown in FIG. 23. During sampling, the kidneys on both sides were isolated, the cortex and inner medulla of each kidney were isolated, and the cortex was stored in trizol. When in use, mRNAs in the cortex were extracted using the trizol method, and the mRNA expressions of AKI biomarkers (KIM-1 and NGAL, with the results shown in FIG. 24) and inflammatory factors (IL-1β, IL-18, TNF-α, and MCP-1, with the results shown in FIG. 25) were detected by the Qpcr method. A tissue lysis buffer was added to homogenize the kidneys by ultrasound, the total protein was then extracted, and the inflammatory factors (p-NFκB/NFκB, pro-IL-1β/ IL-1β) in the renal cortex tissues were detected by western blotting. The results are shown in FIG. 26. The experimental data were statistically analyzed by one-way ANOVA.

FIG. 23 showed that the LPS treatment induced a significant increase in the level of serum creatinine in the mice, and AOS2 significantly reduced the serum creatinine to show the same effect on reducing the serum creatinine as dexamethasone, a drug for anti-inflammatory response as a positive control, suggesting that the alginate disaccharides had a renoprotective effect. Dex represents dexamethasone, * indicates p<0.05 as compared to the CTL group (the control group), and # indicates p<0.05 as compared to the LPS group (the model group). The results showed that compared with the control group, the serum creatinine of the mice significantly increased after intraperitoneal injection of LPS, and the serum creatinine of the mice in the AOS2 administration group decreased significantly and could basically return to a normal level, indicating that the alginate disaccharides had a significant protective effect against the LPS-induced decline of mouse kidney function.

FIG. 24 showed that the LPS treatment induced an increase in the mRNA levels of acute kidney injury (AKI) markers (i.e., renal tubular injury markers) Kim-1 and NGAL in the mouse kidney tissues, suggesting that the alginate disaccharides had a renoprotective effect. * indicates p<0.05 as compared to the CTL group, and # indicates p<0.05 as compared to the LPS group. The results showed that the AKI markers (KIM-1 and NGAL) in the mice significantly increased after intraperitoneal injection of LPS, and the production of KIM-1 and NGAL was significantly reduced after treatment with AOS2, indicating that AOS2 had a significant protective effect against the LPS-induced kidney injury in the mice.

FIG. 25 showed that the LPS treatment induced a significant increase in the inflammatory factors of the mouse kidney tissues, and AOS2 significantly inhibited the inflammatory responses of the kidneys, suggesting that the alginate disaccharides had a renoprotective effect. * indicates p<0.05 as compared to the CTL group, and # indicates p<0.05 as compared to the LPS group. The results showed that the gene expressions of inflammatory factors (IL-1β, IL-18, TNF-α, and MCP-1) in the mice significantly increased after intraperitoneal injection of LPS, and the treatment with AOS2 significantly reduced the production of inflammatory factors, suggesting that the alginate disaccharides had a protective effect against the LPS-induced acute kidney injury in the mice.

FIG. 26 showed that the LPS treatment induced a significant increase in the inflammatory factors of the mouse kidney tissues, and AOS2 significantly inhibited the inflammatory responses of the kidneys, with an effect approaching that of the positive control dexamethasone, suggesting that AOS2 had a renoprotective effect. * indicates p<0.05 as compared to the CTL group, and # indicates p<0.05 as compared to the LPS group. The results showed that after LPS treatment, the protein expressions of renal inflammatory factors such as TLR4, p-NFκB/NFκB, and pro-IL-1p/IL-1β in the mice increased significantly, and the treatment with AOS2 significantly reduced the production of the inflammatory factors, with an effect approaching that of dexamethasone, indicating that AOS2 had a significant anti-inflammatory effect.

### II. Effect of alginate trisaccharides (AOS3) against mouse acute kidney injury induced by LPS

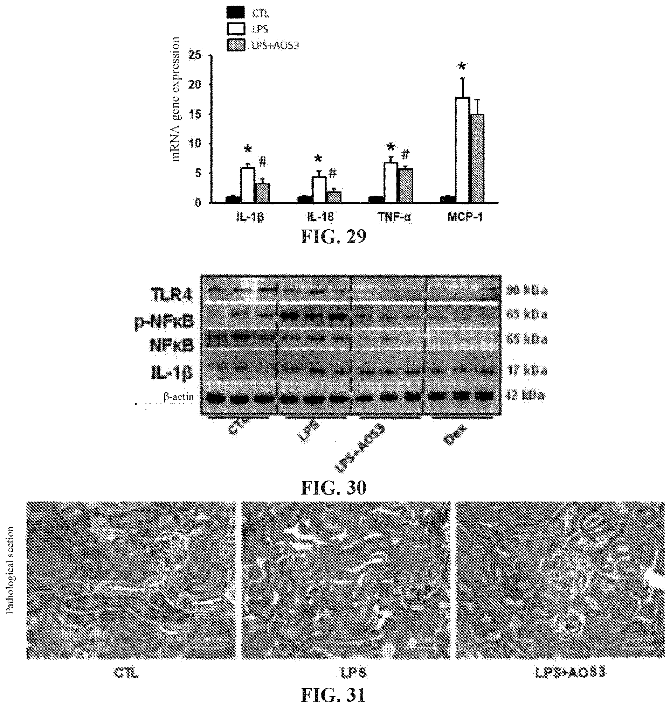

The same experimental method was used as that for the alginate disaccharides. The serum creatinine in the mice was determined by using the creatinine assay kit from Nanjing Jiancheng, with the results shown in FIG. 27. During sampling, the kidneys on both sides were isolated, the cortex and inner medulla of each kidney were isolated, and the cortex was stored in trizol. When in use, mRNAs in the cortex were extracted using the trizol method, and the mRNA expressions of AKI biomarkers (KIM-1 and NGAL, with the results shown in FIG. 28) and inflammatory factors (IL-1β, IL-18, TNF-α, and MCP-1, with the results shown in FIG. 29) were detected by the Qpcr method. A tissue lysis buffer was added to homogenize the kidneys by ultrasound, the total protein was then extracted, and the inflammatory factors (p-NFκB/NFκB, pro-IL-1β/ IL-1β) in the renal cortex tissues were detected by western blotting. The results are shown in FIG. 30. In addition, the animals were sacrificed after the last blood collection, the kidneys were fixed in 4% formaldehyde solution, and subjected to conventional paraffin embedding, sectioning, and HE staining to observe the general kidney tissue morphology under a light microscope. The results are shown in FIG. 31. The experimental data were statistically analyzed by one-way ANOVA.

FIG. 27 showed that the LPS treatment induced a significant increase in the level of serum creatinine in the mice, and AOS3 significantly reduced the serum creatinine to show the same effect on reducing the serum creatinine as dexamethasone, suggesting that the alginate disaccharides had a renoprotective effect. Dex represents dexamethasone, * indicates p<0.05 as compared to the CTL group, and # indicates p<0.05 as compared to the LPS group. The results showed that compared with the control group, the serum creatinine of the mice significantly increased after intraperitoneal injection of LPS, and the serum creatinine of mice in the AOS3 administration group decreased significantly and could basically return to a normal level, suggesting that the alginate trisaccharides had a significant protective effect against the LPS-induced decline of mouse kidney function.

FIG. 28 showed that the LPS treatment induced an increase in the mRNA levels of acute kidney injury (AKI) markers (i.e., renal tubular injury markers) Kim-1 and NGAL in the mouse kidney tissues, suggesting that the alginate trisaccharides had a renoprotective effect. * indicates p<0.05 as compared to the CTL group, and # indicates p<0.05 as compared to the LPS group. The results showed that the AKI markers (KIM-1 and NGAL) in the mice significantly increased after intraperitoneal injection of LPS, and the production of KIM-1 and NGAL was significantly reduced after treatment with AOS3, suggesting that AOS3 had a protective effect against the LPS-induced kidney injury in the mice.

FIG. 29 showed that the LPS treatment induced a significant increase in the inflammatory factors of the mouse kidney tissues, and AOS3 significantly inhibited the inflammatory responses of the kidneys, suggesting that the alginate trisaccharides had a renoprotective effect. * indicates p<0.05 as compared to the CTL group, and # indicates p<0.05 as compared to the LPS group. The results showed that the gene expressions of inflammatory factors (IL-1β, IL-18, TNF-α, and MCP-1) in the mice significantly increased after intraperitoneal injection of LPS, and treatment with AOS3 significantly reduced the production of inflammatory factors, suggesting that the alginate trisaccharides had a convincible protective effect against the LPS-induced acute kidney injury in the mice.

FIG. 30 showed that the LPS treatment induced a significant increase in the inflammatory factors of the mouse kidney tissues, and AOS3 significantly inhibited the inflammatory responses of the kidneys, with an effect approaching that of the positive control dexamethasone, suggesting that AOS3 had a better renoprotective effect. * indicates p<0.05 as compared to the CTL group, and # indicates p<0.05 as compared to the LPS group. The results showed that after LPS treatment, the protein expressions of renal inflammatory factors such as TLR4, p-NFκB/NFκB, and pro-IL-1β/IL-1β in the mice increased significantly, and the treatment with AOS3 significantly reduced the production of the inflammatory factors, with an effect approaching that of dexamethasone, indicating that AOS3 had a convincible anti-inflammatory effect.

FIG. 31 showed the renal histopathological manifestations. Specifically, in the control group, the glomerular morphology, mesangial cells and renal tubules were basically normal; in the LPS group, the glomeruli showed increased volume, the renal interstitium showed inflammatory cell infiltration, the renal tubules were widely dilated, the epithelial cells underwent swelling, necrosis and shedding, and vacuoles were visible; and in the LPS+AOS3 group, the glomeruli and renal tubules showed mild lesions. The results showed that AOS3 had a significant protective effect against the mouse renal morphological changes induced by the treatment with endotoxin phosphorus lipopolysaccharides.

### III. Effect of alginate tetrasaccharides (AOS4) and mixed saccharides against mouse acute kidney injury induced by LPS

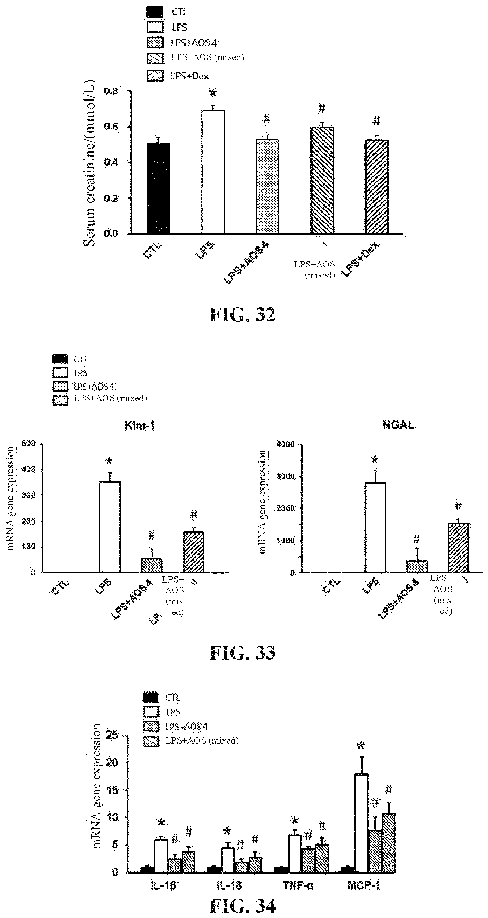

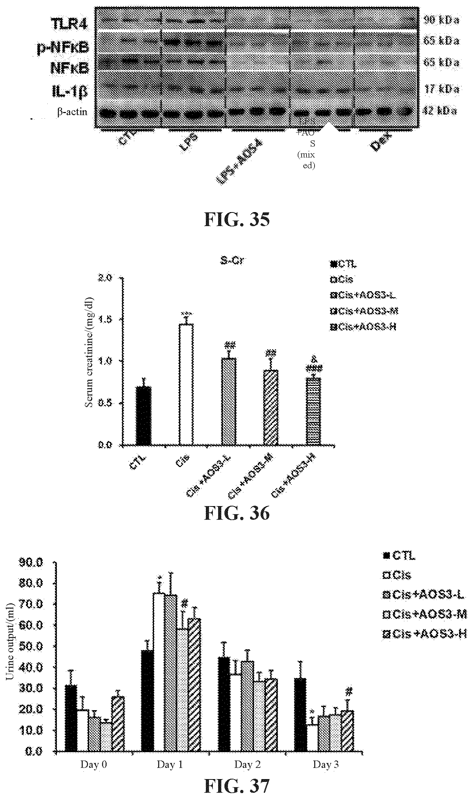

The same experimental method was used as that for the alginate disaccharides and alginate trisaccharides. The mice to be modeled were randomly divided into a control group, a model group, a model + administration group 1 (AOS4 at 0.1 g/kg/day), a model + administration group 2 (mixed alginate oligosaccharides with a degree of polymerization of 2-8 from Ocean University of China, with a mass spectrum shown in FIG. 40, and at a dose of 0.1 g/kg/day) and a model + positive control group (dexamethasone acetate at 0.1 g/kg/day), with 6 mice in each group. The samples were intragastrically administered; the model group and the control group were both fed by gavage with the same volume of normal saline; and dexamethasone was administered by intraperitoneal injection. The serum creatinine in the mice was determined by using the creatinine assay kit from Nanjing Jiancheng, with the results shown in FIG. 32. During sampling, the kidneys on both sides were isolated, the cortex and inner medulla of each kidney were isolated, and the cortex was stored in trizol. When in use, mRNAs in the cortex were extracted using the trizol method, and the mRNA expressions of AKI biomarkers (KIM-1 and NGAL, with the results shown in FIG. 33) and inflammatory factors (IL-1β, IL-18, TNF-α, and MCP-1, with the results shown in FIG. 34) were detected by the Qpcr method. A tissue lysis buffer was added to homogenize the kidneys by ultrasound, the total protein was then extracted, and the inflammatory factors (p-NFκB/NFκB, pro-IL-1β/ IL-1β) in the renal cortex tissues were detected by western blotting. The results are shown in FIG. 35. The experimental data were statistically analyzed by one-way ANOVA.

FIG. 32 showed that the LPS treatment induced a significant increase in the level of serum creatinine in the mice; the mixed saccharides showed certain effect against the decrease in the serum creatinine; and AOS4 significantly reduced the serum creatinine to show an effect approaching that of the dexamethasone on reducing the serum creatinine, suggesting that the alginate oligosaccharides had a renoprotective effect. Dex represents dexamethasone, * indicates p<0.05 as compared to the CTL group, and # indicates p<0.05 as compared to the LPS group. The results showed that compared with the LPS control group, the serum creatinine of the mice significantly increased after intraperitoneal injection of LPS, and the serum creatinine of the mice in the AOS4 + dexamethasone administration group decreased significantly and could basically return to a normal level, indicating that the alginate tetrasaccharides had a significant protective effect approaching that of dexamethasone against the LPS-induced decline of mouse kidney function.

FIG. 33 showed that the LPS treatment induced an increase in the mRNA levels of acute kidney injury (AKI) markers (i.e., renal tubular injury markers) Kim-1 and NGAL in the mouse kidney tissues; and both markers were somewhat reduced by administration of the alginate tetrasaccharides and the mixed saccharides (at a dose of 0.1 g/kg/day), and were significantly reduced in particular by the alginate tetrasaccharides, suggesting that the alginate trisaccharides had a better renoprotective effect. * indicates p<0.05 as compared to the CTL group, and # indicates p<0.05 as compared to the LPS group. The results showed that the AKI markers (KIM-1 and NGAL) in the mice significantly increased after intraperitoneal injection of LPS, and the production of KIM-1 and NGAL was significantly reduced after treatment by administration with AOS4, suggesting that the alginate tetrasaccharides had a protective effect against the LPS-induced kidney injury in the mice.

FIG. 34 showed that the LPS treatment induced a significant increase in the inflammatory factors of the mouse kidney tissues, and the inflammatory responses of the kidneys were significantly inhibited by AOS4 and the mixed saccharides, among which AOS4 showed a better renoprotective effect. * indicates p<0.05 as compared to the CTL group, and # indicates p<0.05 as compared to the LPS group. The results showed that the gene expressions of inflammatory factors (IL-1β, IL-18, TNF-α, and MCP-1) in the mice significantly increased after intraperitoneal injection of LPS, and the production of inflammatory factors was significantly reduced by treatment with AOS4 and the mixed saccharides, among which AOS4 had a protective effect against the LPS-induced acute kidney injury in the mice.

FIG. 35 showed that the LPS treatment induced a significant increase in the inflammatory factors of the mouse kidney tissues, and AOS4 and the mixed saccharides significantly inhibited the inflammatory responses of the kidneys, with an effect approaching that of the positive control dexamethasone, suggesting that AOS4 had a better renoprotective effect. * indicates p<0.05 as compared to the CTL group, and # indicates p<0.05 as compared to the LPS group. The results showed that after LPS treatment, the protein expressions of renal inflammatory factors such as TLR4, p-NFκB/NFκB, and pro-IL-1β/IL-1β in the mice increased significantly, and the treatment with the alginate tetrasaccharides and the mixed saccharides significantly reduced the production of the inflammatory factors, with an effect basically the same as that of dexamethasone, indicating that the alginate tetrasaccharides had a very effective anti-inflammatory effect.

### Experimental conclusion

The kidney injury induced by treatment with endotoxin phosphorus lipopolysaccharides (LPS) is a classical and standard animal model that simulates the acute kidney injury induced by clinical infection. The level of serum creatinine in the LPS-treated mice of the model group increased significantly. After the mice were treated with the alginate disaccharides, alginate trisaccharides and alginate tetrasaccharides at 0.1 g/kg/day, the level of serum creatinine decreased significantly, the levels of kidney injury markers (KIM-1, NGAL) and the expressions of inflammatory factors decreased significantly, and the renal pathological changes were ameliorated significantly. The mixed saccharides with the degree of polymerization of 2-8 also ameliorated the above parameters to varying degrees, but there was still a gap between the mixed saccharides and the alginate disaccharides, alginate trisaccharides and alginate tetrasaccharides. These results indicate that the alginate oligosaccharides with a uniform degree of polymerization have a convincible protective effect against the acute kidney injury induced by endotoxin phosphorus lipopolysaccharides.

### Example 6 Effect of alginate oligosaccharides with uniform degree of polymerization against mouse acute kidney injury induced by cisplatin

Cisplatin-induced kidney injury is a classical and standard animal model that simulates the acute kidney injury induced by drugs that cause nephrotoxicity. With the mouse cisplatin model, we administered the alginate oligosaccharides with different uniform degrees of polymerization and a mixture thereof, and then compared with a blank group and a non-administrated model group to investigate the therapeutic effects of the individual alginate oligosaccharides with a uniform degree of polymerization.

### I. Effect of alginate trisaccharides (AOS3) against mouse acute kidney injury induced by cisplatin

30 male C57BL/J6 mice of 22-28 grams were selected. Before treatment, the 24-hour urine output was collected and measured without abnormalities. The mice to be modeled were randomly divided into the following groups: a control group, a model group, a model + low-dose administration group (AOS3 at 0.05 g/kg/day), a model + medium-dose administration group (AOS3 at 0.1g/kg//day), and a model + high-dose administration group (AOS3 at 0.2 g/kg/day), with 6 mice in each group. The alginate trisaccharides was intragastrically administered, and the model group and the control group were all fed by gavage with the same volume of normal saline. Cisplatin was used to induce the drug toxin-associated acute kidney injury. During modeling, each model group was intraperitoneally injected with cisplatin at 20mg/kg, and the control group was intraperitoneally injected with the same volume of normal saline. Immediately after modeling, the mice were put back into the mouse metabolic cages for observation, during which the weight, food intake, water intake and urine output of the mice were measured (see FIG. 37 for the results). The mice were sacrificed 72 hours later for sampling, and urine was collected. Blood samples were collected from the inferior vena cava of mice, and then centrifuged to collect upper serum. Then, the serum creatinine in the mice was determined by using the creatinine assay kit from Nanjing Jiancheng, with the results shown in FIG. 36. During sampling, the kidneys on both sides were isolated, the cortex and inner medulla of each kidney were isolated, and the cortex was stored in trizol. When in use, mRNAs in the cortex were extracted using the trizol method, and the mRNA expressions of AKI biomarkers (KIM-1 and NGAL, with the results shown in FIG. 38) were detected by the Qpcr method. A tissue lysis buffer was added to homogenize the kidneys by ultrasound, the total protein was then extracted, and the inflammatory factors (p-NFκB/NFκB and IL-1β) in the renal cortex tissues were detected by western blotting. The results are shown in FIG. 39. The experimental data were statistically analyzed by one-way ANOVA.

FIG. 36 showed that the cisplatin treatment induced a significant increase in the level of serum creatinine in the mice, and AOS3 significantly reduced the serum creatinine, suggesting that the alginate trisaccharides had a renoprotective effect. * indicates p<0.05 as compared to the CTL group (the control group), and # indicates p<0.05 as compared to the Cis group (the model group). The results showed that compared with the control group, the serum creatinine of the mice significantly increased after intraperitoneal injection of cisplatin, and the serum creatinine of the mice in the AOS3 administration group decreased significantly and could basically return to a normal level, indicating that the alginate trisaccharides had a significant protective effect against the cisplatin-induced decline of mouse kidney function.

FIG. 37 showed that cisplatin-induced urine output of the mice first increased within 24 h and then decreased within 72 h to enter an oliguric period, and after the administration with AOS3 at different concentrations, the urine output of the mice was first reduced within 24 h to restore the kidney function, and then the urine output gradually became normal, suggesting that the alginate trisaccharides had a renoprotective effect. * indicates p<0.05 as compared to the CTL group, and # indicates p<0.05 as compared to the model group. The results showed that impaired urine concentration occurred in the kidney of mice in the model group after the injection of cisplatin, and the urine output first increased significantly and then entered the oliguric stage. After the treatment with AOS3 at three different doses, the urine output first somewhat decreased and then became normal, suggesting certain recovery of the renal tubular function. Moreover, a certain degree of dose dependence was shown.

FIG. 38 showed that the cisplatin treatment induced an increase in the mRNA levels of acute kidney injury (AKI) markers (i.e., renal tubular injury markers) Kim-1 and NGAL in the mouse kidney tissues, suggesting that the alginate trisaccharides had a renoprotective effect. * indicates p<0.05 as compared to the CTL group, and # indicates p<0.05 as compared to the model group. The results showed that the AKI markers (KIM-1 and NGAL) in the mice significantly increased after intraperitoneal injection of cisplatin, and the production of KIM-1 and NGAL was significantly reduced after treatment with AOS3, indicating that AOS3 had a significant protective effect against the cisplatin-induced kidney injury in the mice.

FIG. 39 showed that the cisplatin treatment induced a significant increase in the inflammatory factors of the mouse kidney tissues, and AOS3 significantly inhibited the inflammatory responses of the kidneys, suggesting that AOS3 had a renoprotective effect. * indicates p<0.05 as compared to the CTL group, and # indicates p<0.05 as compared to the model group. The results showed that after cisplatin treatment, the protein expressions of renal inflammatory factors such as TLR4, p-NFκB/NFκB, and IL-1β in the mice increased significantly, and the treatment with AOS3 significantly reduced the production of the inflammatory factors, indicating that AOS3 had a significant anti-inflammatory effect.

Similarly, further studies found that the alginate disaccharides and the alginate tetrasaccharides showed similar efficacy to the alginate trisaccharides against the cisplatin-induced acute kidney injury in the mice.

### Experimental conclusion

The level of serum creatinine in the cisplatin-treated mice of the model group increased significantly. After the mice were treated with the alginate disaccharides, the alginate trisaccharides and the alginate tetrasaccharides at 0.1 g/kg/day, the level of serum creatinine decreased significantly, and the levels of kidney injury factors (KIM-1 and NGAL) and the expressions of inflammatory factors decreased significantly. The therapeutic effect showed dose dependence. These results indicate that the alginate disaccharides, the alginate trisaccharides and the alginate tetrasaccharides show a convincible protective effect against the cisplatin-induced acute kidney injury.

Finally, it should be noted here that the above only provides some preferred embodiments of the present invention, and cannot be construed as a limitation to the scope of protection of the present invention, and some non-essential improvements and adjustments made by those skilled in the art according to the above description of the present invention should fall within the scope of protection of the present invention.

## Claims

1. Use of an alginate oligosaccharide or a pharmaceutically acceptable salt thereof in preparation of a drug for treatment of acute kidney injury, wherein the alginate oligosaccharide is an alginate disaccharide, an alginate trisaccharide, or an alginate tetrasccharide.

2. The use according to claim 1, wherein the alginate oligosaccharide is composed of monosaccharides G, M and/or Δ linked by glycosidic bonds at positions 1,4; and wherein G represents α-L-guluronic acid, M represents β-D-mannuronic acid, and Δ represents that β-elimination occurs at positions 4,5 of the α-L-guluronic acid or β-D mannuronic acid to generate unsaturated monosaccharides with conjugated double bonds at positions 4,5.

3. The use according to claim 2, wherein the alginate disaccharide is selected from ΔG, ΔM or a combination thereof.

4. The use according to claim 2, wherein the alginate trisaccharide is selected from one or more of ΔGG, ΔGM, ΔMM and ΔMG.

5. The use according to claim 2, wherein the alginate tetrasaccharide is selected from one or more of ΔGGG, ΔGGM, ΔGMG, ΔGMM, ΔMMG, ΔMMM, ΔMGG, and ΔMGM.

6. The use according to claim 1, wherein the pharmaceutically acceptable salt is a sodium salt, a potassium salt, a calcium salt, a magnesium salt, and/or an ammonium salt.

7. The use according to any one of claims 1 to 6, wherein the acute kidney injury is induced by hypoperfusion, infection, or renal toxicity of a drug.

8. An alginate oligosaccharide or a pharmaceutically acceptable salt thereof for treatment of acute kidney injury, wherein the alginate oligosaccharide is an alginate disaccharide, an alginate trisaccharide, or an alginate tetrasaccharide.

9. A method for treating acute kidney injury, comprising administering a therapeutically effective amount of an alginate oligosaccharide or its pharmaceutically acceptable salt to a patient in need thereof, wherein the alginate oligosaccharide is an alginate disaccharide, an alginate trisaccharide, or an alginate tetrasaccharide.
